(19) Europäisches Patentamt
European Patent Office
Office européen des brevets

(11) EP 2 540 690 A1

(12) EUROPEAN PATENT APPLICATION
published in accordance with Art. 153(4) EPC

(43) Date of publication:
02.01.2013 Bulletin 2013/01

(21) Application number: 11747569.9

(22) Date of filing: 23.02.2011

(51) Int Cl.:
*C07B 61/00* (2006.01) *G01N 33/15* (2006.01)

(86) International application number:
PCT/JP2011/054681

(87) International publication number:
WO 2011/105617 (01.09.2011 Gazette 2011/35)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 24.02.2010 JP 2010038412

(71) Applicant: Sumitomo Chemical Company, Limited
Tokyo 104-8260 (JP)

(72) Inventor: NAGAHORI, Hirohisa
Osaka-shi
Osaka 554-0022 (JP)

(74) Representative: Duckworth, Timothy John
J A Kemp
14 South Square
Gray’s Inn
London WC1R 5JJ (GB)

# (54) METHOD FOR PREDICTING ACTIVITY OF CHEMICAL SUBSTANCE

(57) Provided is a prediction method that includes the step of evaluating activity of a chemical substance on the basis of the energy density of a reactive molecular orbital possessed by the constituent molecule of the chemical substance, the orbital corresponding to the kind of activity that is to be predicted.

EP 2 540 690 A1

Printed by Jouve, 75001 PARIS (FR)

## Description

### Technical Field

The present invention relates to a method for predicting activity of a chemical substance, and more specifically, a method for predicting activity of a chemical substance utilizing an index regarding reactivity of the molecule constituting the chemical substance which is calculated from the electron density and the orbital energy level of a reactive molecular orbital possessed by the molecule, and so on.

### Background Art

In development of a chemical substance, for example, a structure of a chemical substance that is to be a substrate is designed on the basis of the reactivity of the molecule constituting the chemical substance that is to be a substrate, such as the structure of a receptor and interaction between the receptor and the substrate, and a molecule having an optimum activity or safety (or toxicity) is selected on the basis of the correlation with the influence exerted on a living body by the molecule.

In the field of synthetic chemistry, for example, a synthetic route of a target chemical substance is designed on the basis of reactivity of molecule(s) constituting Chemical substance(s) that are starting material(s) such as a chemical structure of the molecule constituting the chemical substance that is a starting material and interaction between molecules respectively constituting the plural chemical substances that are starting materials and so on, and a synthetic route having excellent yield or selectivity is selected.

In such designing of a structure of a chemical substance and a synthetic route, it is very important to evaluate the reactivity of the chemical substance in advance, namely to predict activity of the chemical substance, and thus development of an excellent method for achieving this is desired.

Conventionally, for predicting activity of a chemical substance, for example, a prediction method based on the frontier theory, and a prediction method by comparing the magnitude of electron density and so like have been used in combination (Ryousi-Kagaku Nyumon, Revised third edition, Teijiro Yonezawa, 1983, Kabushiki Kaisha Kagakudojin; Japanese Patent Laying-Open No. 2004-12451).

### Disclosure of the Invention

However, in a chemical substance having a complicated structure, since it is sometimes the case that the prediction result obtained by utilizing a prediction method based on the frontier orbital theory, and the prediction result obtained by a prediction method by comparing the magnitude of electron density do not coincide with each other, it is not easy to evaluate the reactivity of the chemical substance in advance.

Concretely, for example, in the prediction method by comparing the magnitude of electron density, since stabilization by delocalization of electrons in a transition state was not taken into account, it was impossible to predict the reactivity for such a reaction that the transition state defines the reactivity of the molecule. On the other hand, the prediction method based on the frontier theory revealed that, in a chemical substance having a simple structure, namely, in a model molecule, the lowest unoccupied molecular orbital (LUMO) is involved in electrophilic reaction and distribution of the unoccupied molecular orbitals defines the reactivity of the molecule, and thereby the method was able to predict the reactivity for the reaction in which the transition state defines the reactivity of the molecule. However, in an actual chemical substance having a complicated structure, it is sometimes the case that the lowest unoccupied molecular orbital is distributed in an atom not involved in electrophilic reaction, and an unoccupied orbital having the second lowest energy or even an unoccupied orbital having the third lowest energy is involved in the reactivity of the molecule, and therefore, the prediction of the reactivity was limited in a reaction such that there is a reactive molecular orbital other than the frontier orbital.

The present invention provides a novel method for predicting activity of a chemical substance utilizing an index of reactivity of a molecule calculated on the basis of a quantum chemical calculation taking into account further a reactive molecular orbital other than the frontier orbital.

Concretely, the present invention provides:

1. A prediction method including the step of evaluating a kind of activity of a chemical substance that is to be predicted, on the basis of an energy density of reactive molecular orbital (ERMO) of the molecule constituting the chemical substance which corresponds to the kind of activity that is to be predicted;
2. A method for predicting activity of a chemical substance including the steps of:

    1) calculating a value of

$$\Sigma i \{(Ei\text{-}Es) * Qi\mu\}$$

wherein Es represents a standard energy level determined in accordance with a kind of activity that is to be predicted, $\alpha$ represents an arbitrary atom in the molecule constituting the chemical substance, i represents an arbitrary molecular orbital possessed by the molecule, Ei represents an orbital energy level of i obtainable by quantum chemical calculation, and Qi$\mu$ represents an electron density at the atom $\alpha$ of the i, and "*" means multiplication;
when the i corresponds to a reactive molecular orbital described in either of the following (1) and (2),
2) setting the calculated value as an energy density of reactive molecular orbital (ERMO), and
3) evaluating the kind of activity of the chemical substance that is to be predicted, on the basis of the set value or a result obtained by statistically analyzing the value (hereinafter, also referred to as the present prediction method):

<Existing reactive molecular orbital>

(1) a molecular orbital at an orbital energy level that is lower than the standard energy level and higher than or equal to the energy level of the lowest unoccupied molecular orbital (ELUMO); and
(2) a molecular orbital at an orbital energy level that is higher than the standard energy level and lower than or equal to the energy level of the highest occupied molecular orbital (EHOMO);

3. A method for predicting activity of a plurality of chemical substances, including both the following (A) and the following (B) (hereinafter, also referred to as the second present prediction method):

(A) a method for predicting activity of at least one chemical substance is the prediction method according to the above 2.; and
(B) a method for predicting activity of at least one chemical substance is a prediction method including the steps of, when an orbital energy level that is equivalent to the standard energy level exists as an arbitrary molecular orbital possessed by the molecule constituting the at least one chemical substance, setting the ERMO at 0 (eV), and evaluating the kind of activity of the chemical substance that is to be predicted, on the basis of the set value or a result obtained by statistically analyzing the value;

4. The prediction method according to either the above 2. or 3, wherein the evaluation step is a step of evaluating the kind of activity of the chemical substance that is to be predicted on the basis of either (i) a difference between a set value and a reference value, (ii) a discrimination score calculated by both a discriminant that is predetermined on the basis of the correlation between a plurality of set values and the kind of activity that is to be predicted, and a target set value, or (iii) a predicted value calculated by both a regression equation that is predetermined on the basis of the correlation between a plurality of set values and the kind of activity that is to be predicted, and a target set value;
5. The prediction method according to the above 4., wherein the discriminant or the regression equation is a linear functional equation represented by:

$$y = a * x + b$$

wherein x is an ERMO minimum value (ERMOmin) or an ERMO maximum value (ERMOmax), wherein the smallest value in the energy densities of reactive molecular orbitals possessed by the molecule is called ERMO minimum value and the largest value is called ERMO maximum value, and "*" means multiplication, a and b represent constants that are predetermined by using a standard chemical substance depending on the kind of activity that is to be predicted;
6. The prediction method according to any one of the above 2. to 5., wherein the arbitrary atom in the molecule constituting the chemical substance is an aromatic halogenated carbon atom, and the kind of activity that is to be predicted is skin sensitization;
7. The prediction method according to any one of the above 2. to 5., wherein the arbitrary atom in the molecule constituting the chemical substance is a carbon atom on an aromatic ring in a phenolic compound, and the kind

of activity that is to be predicted is a radical scavenging effect;

8. The prediction method according to the above 2. or 3., wherein the arbitrary atom in the molecule constituting the chemical substance is a carbon atom on an aromatic ring in a mono-substituted benzenic compound, and the kind of activity that is to be predicted is orientation of nitration; and

9. The prediction method according to the above 2. or 3., wherein the arbitrary atom in the molecule constituting the chemical substance is a carbon atom at ortho or para position in biphenyl, and the kind of activity that is to be predicted is "ortho/para orientation in electrophilic substitution reaction of biphenyl"; and so on.

The present invention makes it possible to provide a method capable of predicting activity of a chemical substance even in a chemical substance having a complicated structure, or the like.

Brief Description of the Drawings

Fig. 1 shows the result in Example 2 confirming the correlation between the ERMO maximum values (ERMOmax) represented on the vertical axis and the radical scavenging effects ($k_{30}/k_3$) represented on the horizontal axis. In Example 2 (the case where the arbitrary atom in the molecule constituting the chemical substance is a carbon atom on an aromatic ring in a phenolic compound, and the kind of activity that is to be predicted is a radical scavenging effect), an energy density of reactive molecular orbital (ERMO) was calculated for every carbon atom on an aromatic ring at a standard energy level of -9 eV for 11 kinds of phenolic compounds, and then the maximum value of the calculated values was selected as an ERMO maximum value.

Fig. 2 shows the result in Example 4 confirming the correlation between the ortho/para orientation represented on the vertical axis and the standard energy levels represented on the horizontal axis. In Example 4 (the case where the arbitrary atom in the molecule constituting the chemical substance is a carbon atom at ortho or para position in biphenyl, and the kind of activity that is to be predicted is "ortho/para orientation in electrophilic substitution reaction of biphenyl"), for evaluating ortho/para orientation in electrophilic substitution reaction of biphenyl (compound U) in Example 3 (namely, formation rate of ortho/para substituted products occurring by electrophilic substitution reaction of biphenyl), (1) an energy density of reactive molecular orbital of the carbon atom at ortho or para position on the aromatic ring (also described as ERMOortho, and ERMOpara, respectively) were calculated, and then ERMOortho/ERMOpara was calculated, and represented on the vertical axis of Fig. 2, and (2) for biphenyl (compound U), standard energy levels -11, -10.5, -10, and -9.5 eV were represented on the horizontal axis of Fig. 2.

Modes for Carrying Out the Invention

In the following, the details of the present invention will be described.

In the present invention, "kind of activity (of chemical substance) that is to be predicted" means physiological activity (concretely, for example, including pharmacological activity, safety (or toxicity) and so on) of a molecule constituting a chemical substance (concretely, for example, a molecule having a specific reactive site and so on), and qualitative characteristics and scientific matters such as reactivity in chemical reaction and so on. Here, "toxicity" includes, for example, acute toxicity, eye irritancy, skin irritancy, sensitization, carcinogenicity, mutagenicity, reproductive toxicity, sub-acute toxicity, chronic toxicity and so on can be recited.

"Activity of (chemical substance)" includes the presence or absence or intensity of physiological activity (concretely, for example, including pharmacological activity, safety (or toxicity) and so on) of a molecule constituting a chemical substance (concretely, for example, a molecule having a specific reactive site and so on), and the kind of a reaction product formed by a chemical reaction and quantitative characteristics and scientific matters such as an yield thereof.

In the present invention, "molecule (constituting a chemical substance)" includes, for example, a molecule having a specific reactive site and so on. As such a molecule, molecules having a structure involved in nucleophilic reaction, electrophilic reaction or radical reaction can be preferably recited, and concretely, for example, molecules having an $\alpha,\beta$-unsaturated ketone, a halogenated aromatic ring, an aldehyde and so on can be recited.

"(Arbitrary) atom (in a molecule constituting a chemical substance)" includes, for example, an atom existing in a specific reactive site and so on. As such an atom, atoms actually involved in nucleophilic reaction, electrophilic reaction or radical reaction can be preferably recited, and concretely, such an atom that binds and changes after the reaction when a chemical substance having a reactive structure is compared with a product after the reaction, can be recited.

The present prediction method is a method for predicting activity of a chemical substance, and includes:

1) calculating a value of

$$\Sigma i \{(Ei\text{-}Es) * Qi\mu\}$$

wherein Es represents a standard energy level determined in accordance with a kind of activity that is to be predicted, $\alpha$ represents an arbitrary atom in the molecule constituting the chemical substance, i represents an arbitrary molecular orbital possessed by the molecule, Ei represents an orbital energy level of i obtainable by a quantum chemical calculation, and Qi$\mu$ represents an electron density at the atom $\alpha$ of the i, and "*" means multiplication,
when the i corresponds to a reactive molecular orbital described in either of the following (1) and (2),
2) setting the calculated value as an energy density of reactive molecular orbital (ERMO), and
3) evaluating the kind of activity of the chemical substance that is to be predicted, on the basis of the set value or a result obtained by statistically analyzing the value.

<Existing reactive molecular orbital>

(1) a molecular orbital at an orbital energy level that is lower than the standard energy level and higher than or equal to the energy level of the lowest unoccupied molecular orbital (ELUMO)
(2) a molecular orbital at an orbital energy level that is higher than the standard energy level and lower than or equal to the energy level of the highest occupied molecular orbital (EHOMO).

In the present invention, "quantum chemical calculation" means solving the Schroedinger equation that describes the spatial distribution of each electron in the molecule constituting the chemical substance by using one-electron approximation, to determine orbital energy levels of all molecular orbitals and a coefficient of an atomic orbital. An orbital energy level of every molecular orbital and a coefficient of an atomic orbital of an arbitrary atom in every molecular orbital may be determined, for example, by using ordinary methods such as an empirical molecular orbital method, a semi-empirical molecular orbital method such as an AM1 Hamiltonian method, and a first principal molecular orbital calculation method and so on.

From the result determined by conducting such a quantum chemical calculation (namely, the orbital energy levels of molecular orbitals and the coefficient of an atomic orbital), further, electron density, a lowest unoccupied molecule orbital and an energy level of the lowest unoccupied molecular orbital (ELUMO) and a highest unoccupied molecule orbital and an energy level of the highest occupied molecular orbital (EHOMO) can be calculated by using an ordinary method such as a method as will be described below.

For example, for determining "orbital energy level (of molecular orbital)" and "coefficient of atomic orbital", a method of solving the expressions shown by (A) to (C) below that are LCAO approximated by a linear combination of an atomic orbital function $\chi_\mu$ of an atom constituting a molecular orbital, by a quantum chemical calculation may be used.

(A): $\Psi = \Psi_1\Psi_2 \cdots \Psi_n$
(B): $H_i\Psi_i = E_i\Psi_i$
(C): $\Psi_i = \Sigma_\mu Ci\mu\chi\mu$

wherein "$\Psi$" represents a wave function, "$\Psi$i" represents a molecular orbital function, "Hi" represents a Hamilton function for the molecular orbital $\Psi_i$, $\chi_\mu$ represents an atomic orbital function, $C_{i\mu}$ represents a coefficient of the $\mu$-th atomic orbital in the i-th molecular orbital, and "Ei" represents an orbital energy level in the i-th molecular orbital.

For solving the above approximated formula by a quantum chemical calculation, calculation may be conducted by the AM1 Hamiltonian method among the calculation methods loaded in Hyperchem 8 (Hypercube Inc., FL, USA) that is a commercially available program. In this case, after inputting a structure of a chemical substance in the commercially available program, Hyperchem 8, first (1) a three-dimensional structure is optimized, and then (2) molecular orbital energy levels of all molecular orbitals including energy of the lowest unoccupied molecular orbital (ELUMO) and energy of the highest occupied molecular orbital (EHOMO), and a coefficient of an atomic orbital of an arbitrary atom in every molecular orbital may be calculated.

The "orbital energy level" in the present prediction method means a value of energy of a molecular orbital describing the condition of each electron constituting the molecule constituting the chemical substance (one-electron wave function describing a spatial distribution of each electron in a molecule), and as mentioned above, it may be calculated by a quantum chemical calculation. The "orbital energy level of (arbitrary) molecular orbital (possessed by a molecule)" (Ei) means an orbital energy level of an arbitrary molecular orbital possessed by a molecule constituting the chemical substance and containing an arbitrary atom $\alpha$.

The "standard energy level (determined according to the kind of activity that is to be predicted)" (Es) in the present prediction method means the energy level of the boundary that separates the reactive molecular orbital from other molecular orbitals. The standard energy level is usually the value determined empirically or from the result of statistical analysis of an experimental result, and when a molecule that is to be a reaction partner is determined,

(1) in nucleophilic reaction, it may be set at an energy level higher by a certain energy than the energy level of the highest occupied molecular orbital (EHOMO) of the molecule that is to be a reaction partner; or
(2) in electrophilic reaction, it may be set at an energy level lower by a certain energy than the energy level of the lowest unoccupied molecular orbital (ELUMO) of the molecule that is to be a reaction partner.

When the molecule that is to be a reaction partner is not determinable, or when the certain energy as described above is intended to be optimized, first, after setting tentative several standard energy levels (for example, setting the standard energy levels varying by 0.1 eV from -2 eV), a tentative energy density of reactive molecular orbital is calculated by using a respective one of the tentative standard energy levels for several molecules having known activity, and then the correlation between the activity and the tentative energy density of reactive molecular orbital is determined. The tentative standard energy showing the highest correlation determined may be selected and set as the optimum standard energy level.

The "highest occupied molecular orbital" in the present prediction method means the molecular orbital having the highest energy level among the molecular orbitals occupied by electrons, and is generally called "HOMO". In the frontier orbital theory, it is told that the part having the highest probability density of HOMO is a reacting point of a nucleophile.

The "energy level of the highest occupied molecular orbital" in the present prediction method means the orbital energy level of the highest occupied molecular orbital, and is generally called EHOMO. For determining EHOMO, as described above, for example, calculation may be conducted by using the AM1 Hamiltonian method among the calculation methods loaded in Hyperchem 8 (Hypercube Inc., FL, USA) that is a commercially available program.

The "lowest unoccupied molecular orbital" in the present prediction method means a molecular orbital having the lowest energy level among the molecular orbitals that are not occupied by electrons, and is generally called "LUMO". In the frontier orbital theory, it is told that the part having the highest probability density of LUMO is a reacting point of a nucleophile. For determining ELUMO, as described above, for example, calculation may be conducted by using the AM1 Hamiltanian method among the calculation methods loaded in Hyperchem 8 (Hypercube Inc., FL, USA) that is a commercially available program.

The "reactive molecular orbital" in the present prediction method means a molecular orbital having a possibility of being involved in nucleophilic reaction or electrophilic reaction, and is determined by comparing the orbital energy level calculated by a quantum chemical calculation and the standard energy level. As described above, the "(arbitrary) molecular orbital (possessed by the molecule)" (i) means a specific reactive molecular orbital corresponding to:

(1) a molecular orbital at an orbital energy level that is lower than the standard energy level and higher than or equal to the energy level of the lowest unoccupied molecular orbital (ELUMO); or
(2) a molecular orbital at an orbital energy level that is higher than the standard energy level and lower than or equal to the energy level of the highest occupied molecular orbital (EHOMO),

in arbitrary molecular orbitals possessed by the molecule constituting the chemical substance and containing the arbitrary atom $\alpha$.

In the case of determining a reactive molecular orbital,

(1) for determining a nucleophilic reactive molecular orbital, the specific reactive molecular orbital may be a molecular orbital at an orbital energy level lower than the standard energy level and higher than or equal to the energy level of the lowest unoccupied molecular orbital (ELUMO), and on the other hand,
(2) for determining an electrophilic reactive molecular orbit, the specific reactive molecular orbital may be a molecular orbital at an orbital energy level higher than the standard energy level and lower than or equal to the energy level of highest occupied molecular orbital (EHOMO).

The "electron density" in the present prediction method means density of the molecular orbital describing the state of each electron constituting the molecule (one-electron wave function describing spatial distribution of each electron in the molecule). When there is no electron on the molecular orbital, it is also called orbital density. The electron density may be calculated from the coefficient (C) of the atomic orbital determined by a quantum chemical calculation.

The "electron density at the atom $\alpha$ of the i" in the present prediction method ($Qi\mu$) means the probability that the specific reactive molecular orbital (i) is distributed in the atom $\alpha$. Concretely, "electron density at the atom $\alpha$ of the

i" can be calculated by a calculation formula represented by

$$2 * (C_{i\mu})^2$$

wherein $\mu$ represents the atomic number of the atom $\alpha$, $C_{i\mu}$ represents the coefficient of the $\mu$-th atomic orbital in the i-th molecular orbital, and "*" means multiplication.

The "energy density of reactive molecular orbital (ERMO)" in the present prediction method means an index regarding reactivity of the molecule at the atom $\alpha$.

When the i, that is, (arbitrary) molecular orbital (possessed by the molecule constituting the chemical substance) corresponds to any one of the reactive molecular orbitals described below, the value (ERMO) may be obtained as described above, by calculating the value of

$$\Sigma i\{(Ei\text{-}Es) * Qi\mu\}$$

wherein "*" means multiplication ;

(1) a molecular orbital at an orbital energy level that is lower than the standard energy level and higher than or equal to the energy level of the lowest unoccupied molecular orbital (ELUMO); or
(2) a molecular orbital at an orbital energy level that is higher than the standard energy level and lower than or equal to the energy level of highest occupied molecular orbital (EHOMO).

When there is no reactive molecular orbital as the arbitrary molecular orbital possessed by the molecule constituting the chemical substance, 0 (eV) may be set as ERMO.

By evaluating the kind of activity of the chemical substance that is to be predicted on the basis of the value thus calculated and set (namely, ERMO) or a result obtained by statistically analyzing the value, it is possible to predict activity of the chemical substance.

In the present prediction method, as a technique for "evaluating the kind of activity of the chemical substance that is to be predicted on the basis of the value thus calculated and set (namely, ERMO) or a result obtained by statistically analyzing the value", for example, a technique used in ordinary structure activity relationship or the like may be used.

The "statistical analysis" in the present prediction method means numerically analyzing plural data and estimating the correlations such as the magnitude, variance, probability or causal relationship of the phenomenon and the like, and for example, estimation may be achieved by using an analytical technique of test, estimation, analysis of variance, multiple classification analysis and so on. As a multiple classification analysis, for example, a regression analysis such as a single regression analysis, a multiple regression analysis, a logistic regression analysis or a discrimination analysis and so on may be recited.

The "regression analysis" in the present prediction method is a statistical analysis technique using a regression equation representing the relation between plural data of interest (independent variable) and plural data intended to be described (dependent variable). For example, a regression equation may be calculated by using a least-squares analysis or the like. The "discrimination analysis" in the present prediction method is a statistical analysis technique using a discriminant for classifying plural data into plural categories. For example, a discriminant may be calculated by using Mahalanobis distance or the like.

The evaluation step in the present prediction method may be a step of evaluating the kind of activity of the chemical substance that is to be predicted on the basis of either, for example, (i) a difference between a set value and a reference value, (ii) a discrimination score calculated by both a discriminant that is predetermined on the basis of the correlation between a plurality of set values and the kind of activity that is to be predicted, and a target set value, or (iii) a predicted value calculated by both a regression equation that is predetermined on the basis of the correlation between a plurality of set values and the kind of activity that is to be predicted, and a target set value.

As the discriminant in the above (ii), concretely, for example, a linear functional equation:

$$y = a * x + b$$

wherein, x is the ERMO minimum value (ERMOmin) wherein the smallest value of the energy density of reactive molecular orbital possessed by the molecule is called an ERMO minimum value (ERMOmin), and "*" means multiplication, a and

b represent constants that are predetermined by using a standard chemical substance depending on the kind of activity that is to be predicted can be recited. Based on the discrimination score (y) calculated by such a linear functional equation, the presence or absence of the kind of activity of the chemical substance that is to be predicted may be determined, and further, the level of activity may be determined depending on the magnitude of the difference.

As the regression equation in the above (iii), concretely, for example, a linear functional equation

$$y = a * x + b$$

wherein, x is the ERMO maximum value (ERMOmax) wherein the largest value of the energy density of reactive molecular orbital possessed by the molecule is called an ERMO maximum value, and "*" means multiplication, a and b represent constants that are predetermined by using a standard chemical substance depending on the kind of activity that is to be predicted can be recited. Based on the predicted value (y) calculated by such a linear functional equation, the presence or absence of the kind of activity of the chemical substance may be determined, and further, the level of activity may be determined depending on the magnitude of the predicted value.

From these determination results, by the present prediction method, it becomes possible to evaluate reactivity of the chemical substance in advance, or to predict activity of the chemical substance in designing the structure of the chemical substance or in designing a synthetic route.

As a concrete example, when the arbitrary atom in the molecule constituting the chemical substance is an aromatic halogenated carbon atom, and the kind of activity that is to be predicted is skin sensitization, the present prediction method can be applied. Standard energy level Es in this case may be, for example, 0 (eV).

Then, in the evaluation step of the present prediction method, constant a in the linear functional equation of the discriminant is -5.2, and constant b is -4.5, and when the discrimination score (y) is plus, it may be evaluated that skin sensitization of the chemical substance is positive, whereas when the discrimination score (y) is minus, it may be evaluated that skin sensitization of the chemical substance is negative.

As another concrete example, when the arbitrary atom in the molecule constituting the chemical substance is a carbon atom on an aromatic ring in a phenolic compound, and the kind of activity that is to be predicted is a radical scavenging effect, the present prediction method may be applied. Standard energy level Es in this case may be, for example, -9 (eV).

Then, in the evaluation step of the present prediction method, a predicted value (y) may be calculated from an ERMO maximum value of the compound for that activity is to be predicted by assigning $4 \times 10^{-5}$ to constant a, and 0.00273 to constant b in the linear functional equation of the regression equation, and the calculated predicted value may be evaluated as an index for the radical scavenging effect.

As another concrete example, when the arbitrary atom in the molecule constituting the chemical substance is a carbon atom on an aromatic ring in a mono-substituted benzenic compound, and the kind of activity that is to be predicted is orientation of nitration, the present prediction method can be applied. Standard energy level Es in this case may be set at -10.7 (eV), for example, and carbon atoms on an aromatic ring of the molecule may be evaluated as having orientation of nitration in descending order of ERMO.

As another concrete example, also in the case where the arbitrary atom in the molecule constituting the chemical substance is a carbon atom at ortho or para position in biphenyl, and the kind of activity that is to be predicted is "ortho/para orientation in electrophilic substitution reaction of biphenyl", the present prediction method can be applied.

When there are a plurality of chemical substances for that activity is intended to be predicted, activities of the plural chemical substances can also be predicted efficiently by applying the present prediction method to each of the plural chemical substances, and making general evaluation by combining the evaluation results that are individually obtained, or by applying the second present prediction method.

Also, after preliminarily constructing a correlation equation on the basis of the correlation between the activity of the chemical substance predicted by the present prediction method and ERMO used for the prediction, ERMO possessed by the molecule constituting the chemical substance for which prediction is intended to be newly made is assigned to the correlation equation, and for example, from a discrimination score calculated in this manner, activity of a chemical substance may be predicted.

As concrete application examples of the present invention, the following embodiments of the present invention can be recited.

Examples

Example 1 (the case where the arbitrary atom in the molecule constituting the chemical substance is an aromatic halogenated carbon atom, and the kind of activity that is to be predicted is skin sensitization)

For the following eight kinds of chemical substances having an aromatic halogenated carbon (namely, halogenated aromatic compounds), by assigning the energy of the lowest unoccupied molecular orbital (ELUMO) and a minimum value (ERMOmin) of ERMO that is a value of an energy density of reactive molecular orbital (ERMO) at standard energy level of 0 eV to the discriminant:

$$y = -5.2 \times \mathrm{ERMOmin} - 4.5$$

that is calculated from Mahalanobis distance, a discrimination score (y) was calculated, and activity of the sensitization was evaluated by evaluating in such criteria that (1) the sensitization is positive when the score is plus, and (2) the sensitization is negative when the score is minus. Since a halogenated aromatic compound acts as an electrophilic agent for protein as a reaction partner, and halogen substitution occurs, ELUMO and ERMO in the aromatic halogenated carbon were determined by a quantum chemical calculation while the standard energy was set at 0 eV, and the reactive molecular orbital was set at a molecular orbital at an orbital energy level of lower than or equal to 0 eV and higher than or equal to the energy level of the lowest unoccupied molecular orbital (ELUMO).
The result is shown in Table 1 (ELUMO, ERMO, discrimination score (y), activity evaluation (sensitization) and sensitization (on the basis of those described in reference documents) are recited for each of the chemical substances). The aforementioned energy of the lowest unoccupied molecular orbital (ELUMO) and energy density of reactive molecular orbital (ERMO) were calculated using the AM1 Hamiltonian method using the program Hyperchem 8. As to the presence or absence of sensitization of the chemical substance, those described in the following Reference documents 1, 2 and 3 are shown together in Table 1.
Reference document 1: Patlewicz G. et al., Chem. Res. Toxicol. 21, 521, 2008
Reference document 2: Roberts D. W., Chem. Res. Toxicol. 8, 545, 1995
Reference document 3: Tomlin C. D. S., The Pesticide Manual (Eleventh Edition), British Crop Protection Council, UK, 1997

Compound A: 1-chloro-2, 4-dinitrobenzene (CAS No. 97-00-7)

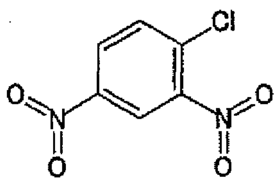

Compound B: 2, 4, 6-trichloro-1, 3, 5-triazine (CAS No. 108-77-0)

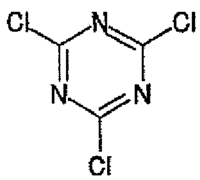

Compound C: 1, 5-Dichloro-2, 4-dinitrobenzene (CAS No. 3698-83-7)

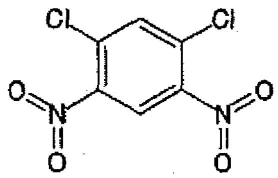

Compound D: 2, 4, 6-Trinitrochlorobenzene (CAS No. 88-88-0)

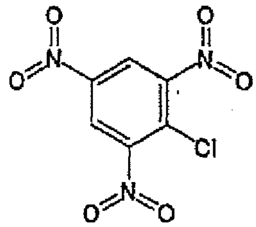

Compound E: 4-chloronitrobenzene (CAS No. 100-00-5)

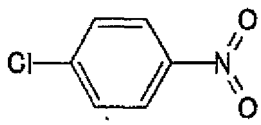

Compound F: brodifacoum (CAS No. 56073-10-0)

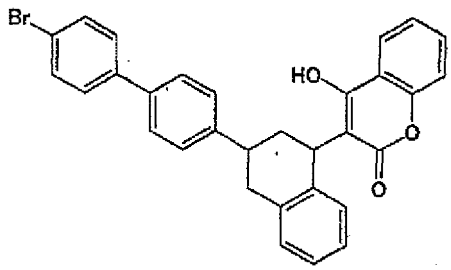

Compound G: chlorsulfuron (CAS No. 64902-72-3)

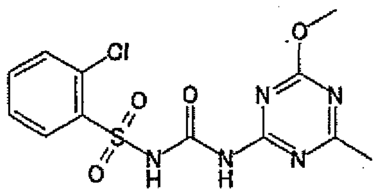

Compound H: sulcofuron-sodium (CAS No. 3567-25-7)

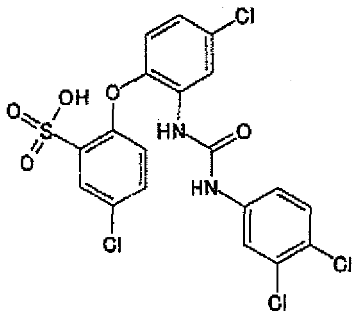

[Table 1]

| Chemical substance | ELUMO | ERMO | Discrimination score (y) | Activity evaluation (sensitization) | Sensitization (on the basis of description of Reference document) |
|---|---|---|---|---|---|
| Compound A | -2.11 | -1.55 | 3.6 | Positive | P |
| Compound B | -1.33 | -1.18 to -1.23 | 1.9 | Positive | P |
| Compound C | -2.29 | -1.61 to -1.68 | 4.2 | Positive | P |
| Compound D | -2.63 | -2.64 | 9.2 | Positive | P |
| Compound E | -1.40 | -0.70 | -0.9 | Negative | N |
| Compound F | -1.09 | -0.06 | -4.2 | Negative | N |
| Compound G | -1.13 | -0.44 | -2.2 | Negative | N |
| Compound H | -1.68 | -0.18 to -0.61 | -1.3 | Negative | N |

As is apparent from Table 1, it was revealed that the sensitization is positive when the discrimination score (y) is plus, and the sensitization is negative when the score is minus. Therefore, it was confirmed that evaluation of activity regarding sensitization predicted by the present prediction method perfectly coincides with those described in the reference documents mentioned above.
It was confirmed that the present invention makes it possible to predict activity of a chemical substance.

Example 2 (the case where the arbitrary atom in the molecule constituting the chemical substance is a carbon atom on an aromatic ring in a phenolic compound, and the kind of activity that is to be predicted is a radical scavenging effect)

For the following eleven kinds of phenolic compounds, an energy density of reactive molecular orbital (ERMO) of every carbon atom on an aromatic ring was calculated while the standard energy level was set at -9 eV, and among the values, the largest value was regarded as an ERMO maximum value (ERMOmax). Fig. 1 shows the correlation between the ERMO maximum value (ERMOmax) represented on the vertical axis, and the radical scavenging effect ($k_{30}/k_3$) represented on the horizontal axis confirmed by a least-squares analysis.
The above energy density of reactive molecular orbital (ERMO) was calculated by using the AM1 Hamiltonian method using the program Hyperchem 8. For the radical scavenging effect ($k_{30}/k_3$) of various phenols, those described in the following Reference document 4 were used.
Reference document 4: Automatic Oxidization (Chemical Mechanism and Application), translated by Kei Matsuzaki, and Zenjiro Osawa, MARUZEN, p159, 1972

Compound I: phenol (CAS No. 108-95-2)

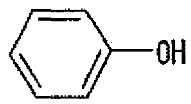

Compound J: p-cresol (CAS No. 106-44-5)

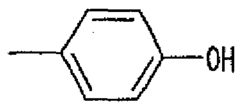

Compound K: p-t-butylphenol

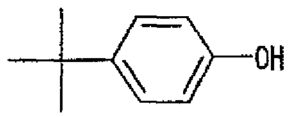

Compound L: m-cresol (CAS No. 108-39-4)

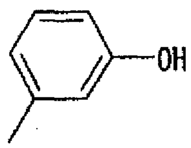

Compound M: o-cresol (CAS No. 95-48-7)

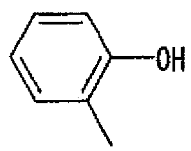

Compound N: 2, 4-dimethylphenol (CAS No. 105-67-9)

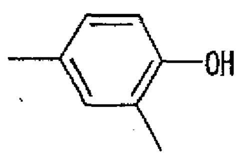

Compound O: Mesitol (CAS No. 527-60-6)

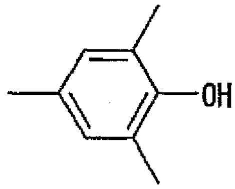

Compound P: dibutylhydroxytoluene (CAS No. 128-37-0)

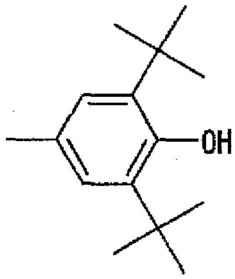

Compound Q: α-naphthol (CAS No. 90-15-3)

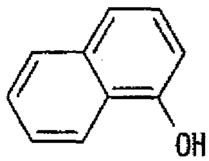

Compound R: β-naphthol (CAS No. 135-19-3)

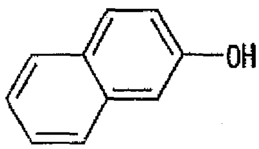

Compound S: 1-Phenanthrol (CAS No. 2433-56-9)

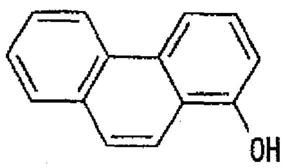

Fig. 1 reveals that there is a positive correlation between the ERMO maximum value (ERMOmax) and the radical scavenging effect. In this manner, the regression equation

$$y = 4 \times 10^{-5} x + 0.0273 \text{ (constant a} = 4 \times 10^{-5}\text{, constant b} = 0.0273\text{)}$$

was obtained. It is expected that the radical scavenging effect can be accurately predicted from an expected value calculated from the regression equation and a target set value, and it is expected to be useful for development of an antioxidant.

Example 3 (the case where the arbitrary atom in the molecule constituting the chemical substance is a carbon atom on an aromatic ring in a mono-substituted benzenic compound, and the kind of activity that is to be predicted is orientation of nitration)

For the positions where the following four kinds of mono-substituted benzenic compounds are nitrated, the standard energy level was set at -10. 7eV, and values of energy density of reactive molecular orbital (ERMO) of carbon atoms at ortho, meta and para positions on an aromatic ring were calculated, and energy densities of reactive molecular orbitals (ERMO) of carbon atoms at ortho, meta and para positions on an aromatic ring were calculated. Among these values, the largest value was regarded as the one having the highest orientation of nitration (this may be set as a reference value), and evaluation was made such that the higher the value, the more the nitration is prone to occur (higher activity).

Since a mono-substituted benzenic compound is converted into a nitrobenzenic derivative by an electrophilic agent ($NO_2^+$), the standard energy was set at -10.7 eV, and the reactive molecular orbital was set at a molecular orbital at an orbital energy level higher than or equal to -10.7 eV and lower than or equal to the energy level of the highest occupied molecular orbital (EHOMO), and ERMO was determined by a quantum chemical calculation.

The result is shown in Table 3 (ERMO, activity evaluation (ranking) and generation rate and ranking of orientation of substituents according to reference documents recited for each of the chemical substances).

The aforementioned energy density of reactive molecular orbital (ERMO) was calculated by using the AM1 Hamiltonian method using the program Hyperchem 8. As to the generation rate of substituent of a chemical substance, those described in Reference document 5 below are shown together in Table 3.
Reference document 5: I. Fleming, Introduction to Frontier Orbital Theory, 1978, Kodansha Scientific

Compound T: anisole (CAS No. 100-66-3)

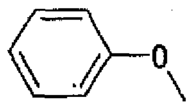

Compound U: biphenyl (CAS No. 92-52-4)

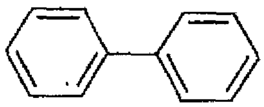

Compound V: ethyl benzoate (CAS No. 93-89-0)

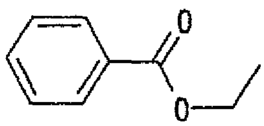

Compound W: nitrobenzene (CAS No. 98-95-3)

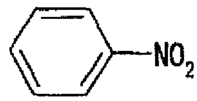

[Table 2]

| | | Ortho position | Meta position | Para position |
|---|---|---|---|---|
| Compound T | ERMO | 0.91 | 0.62 | 0.93 |
| | Ranking of orientation | 2 | 3 | 1 |
| | Generation rate of substituent according to Reference document (ranking of orientation) | 17% (2) | - (3) | 83% (1) |
| Compound U | ERMO | 0.76 | 0.59 | 0.72 |
| | Ranking of orientation | 1 | 3 | 2 |
| | Generation rate of substituent according to Reference document (ranking of orientation) | 53% (1) | - (3) | 47% (2) |
| Compound V | ERMO | 0.49 | 0.50 | 0.42 |
| | Ranking of orientation | 2 | 1 | 3 |
| | Generation rate of substituent according to Reference document (ranking of orientation) | 28% (2) | 68% (1) | 3% (3) |
| Compound W | ERMO | 0.012 | 0.018 | 0.00 |
| | Ranking of orientation | 2 | 1 | 3 |
| | Generation rate of substituent according to Reference document (ranking of orientation) | 6% (2) | 93% (1) | 0.25% (3) |

As is apparent from Table 2, it was confirmed that the ranking of orientation evaluated from ERMO perfectly coincides with the "generation rate of substituent (ranking of orientation)" described in Reference document 5.

In this way, it was confirmed that a substitution position in a mono-substituted benzenic compound can be accurately predicted by the present invention.

Example 4 (the case where the arbitrary atom in the molecule constituting the chemical substance is a carbon atom at ortho or para position in biphenyl, and the kind of activity that is to be predicted is "ortho/para orientation in electrophilic substitution reaction of biphenyl")

For evaluating ortho/para orientation in electrophilic substitution reaction of biphenyl (Compound U) in Example 3 (namely, generation rate of ortho substituent/para substituent generated in electrophilic substitution reaction of biphenyl), (1) after calculating energy densities of reactive molecular orbital of carbon atoms at ortho and para positions on an aromatic ring (respectively, also denoted by ERMOortho, and ERMOpara), ERMOortho/ERMOpara was further calculated, and represented on the vertical axis, while on the other hand, (2) for biphenyl (Compound U), the standard energy level was set at -11, -10.5, -10, and -9.5 eV, and represented on the horizontal axis, and the correlation therebetween was confirmed.

Since an ortho substituent and/or a para substituent is generated from biphenyl depending on the electrophilic agent, ERMO was determined by a quantum chemical calculation while the reactive molecular orbital was regarded as a molecular orbital at an orbital energy level of higher than or equal to the standard energy and lower than or equal to the energy level of the highest occupied molecular orbital (EHOMO).

The result is shown in Fig. 2. The energy density of reactive molecular orbital (ERMO) was calculated by using the AM1 Hamiltonian method using the program Hyperchem 8.

As is apparent from Fig. 2, when the standard energy is increased, the numerical value of the ERMOortho/ERMOpara decreases, so that it was expected that activity of the carbon atom at para position is higher than the activity of the carbon atom at ortho position. High standard energy means a small number of reactive orbitals, and means substituting biphenyl using an electrophilic agent having lower reactivity. That is, it was predicted that the generation rate of para substituent is higher by using an electrophilic agent having lower reactivity. It was confirmed that this prediction result perfectly coincides with the experimental result that "the generation rate of para substituent is high when the electrophilic agent is soft (low reactivity)", described in Reference document 4.

As described above, it was confirmed that in electrophilic substitution reaction of biphenyl, it is possible to evaluate ortho/para generation rate in electrophilic substitution reaction of biphenyl and to predict the ortho/para orientation in the reaction, on the basis of the energy density of reactive molecular orbital (ERMO) depending on the reactivity of the used electrophilic agent, by the present prediction method.

Industrial Applicability

In development of chemical substances, the present invention is used in selecting a molecule having an optimum activity or safety (or toxicity) or in selecting a synthetic route having excellent yield or selectivity in the field of synthetic chemistry.

## Claims

1. A method for predicting activity of a chemical substance, comprising the step of: evaluating a kind of activity of the chemical substance that is to be predicted, on the basis of an energy density of reactive molecular orbital (ERMO) of the molecule constituting the chemical substance which corresponds to the kind of activity that is to be predicted.

2. A method for predicting activity of a chemical substance comprising the steps of:

    1) calculating a value of

$$\Sigma i \{(Ei\text{-}Es) * Qi\mu\}$$

    wherein Es represents a standard energy level determined in accordance with a kind of activity that is to be predicted, $\alpha$ represents an arbitrary atom in the molecule constituting the chemical substance, i represents an arbitrary molecular orbital possessed by said molecule, Ei represents an orbital energy level of i obtainable by quantum chemical calculation, and Qi$\mu$, represents an electron density at said atom $\alpha$ of said i, and "*" means multiplication,
    when said i corresponds to a reactive molecular orbital described in either of the following (1) and (2),
    2) setting the calculated value as an energy density of reactive molecular orbital (ERMO), and
    3) evaluating the kind of activity of the chemical substance that is to be predicted, on the basis of the set value or a result obtained by statistically analyzing the value: <Existing reactive molecular orbital>

        (1) a molecular orbital at an orbital energy level that is lower than said standard energy level and higher than or equal to the energy level of the lowest unoccupied molecular orbital (ELUMO); and
        (2) a molecular orbital wherein said molecular orbital is at an orbital energy level that is higher than said standard energy level and lower than or equal to the energy level of the highest occupied molecular orbital (EHOMO).

3. A method for predicting activity of a plurality of chemical substances, comprising both the following (A) and the following (B):

    (A) a method for predicting activity of at least one chemical substance is the prediction method according to claim 2.; and
    (B) a method for predicting activity of at least one chemical substance is a prediction method including the steps of, when an orbital energy level that is equivalent to the standard energy level exists as an arbitrary molecular orbital possessed by the molecule constituting the at least one chemical substance, setting the ERMO at 0 (eV), and evaluating the kind of activity of the chemical substance that is to be predicted, on the basis of the set value

or a result obtained by statistically analyzing the value.

4. The prediction method according to claim 2 or 3, wherein the evaluation step is a step of evaluating the kind of activity of the chemical substance that is to be predicted on the basis of either (i) a difference between a set value and a reference value, (ii) a discrimination score calculated by both a discriminant that is predetermined on the basis of the correlation between a plurality of set values and the kind of activity that is to be predicted, and a target set value, or (iii) a predicted value calculated by both a regression equation that is predetermined on the basis of the correlation between a plurality of set values and the kind of activity that is to be predicted, and a target set value.

5. The prediction method according to claim 4, wherein said discriminant or said regression equation is a linear functional equation represented by:

$$y = a * x + b$$

wherein x is an ERMO minimum value (ERMOmin) or an ERMO maximum value (ERMOmax) wherein the smallest value in the energy densities of reactive molecular orbitals possessed by said molecule is called ERMO minimum value and the largest value is called ERMO minimum value or ERMO maximum value, and "*" means multiplication, a and b represent constants that are predetermined by using a standard chemical substance depending on the kind of activity that is to be predicted.

6. The prediction method according to any one of claims 2 to 5, wherein the arbitrary atom in the molecule constituting the chemical substance is an aromatic halogenated carbon atom, and the kind of activity that is to be predicted is skin sensitization.

7. The prediction method according to any one of claims 2 to 5, wherein the arbitrary atom in the molecule constituting the chemical substance is a carbon atom on an aromatic ring in a phenolic compound, and the kind of activity that is to be predicted is a radical scavenging effect.

8. The prediction method according to claim 2 or 3, wherein the arbitrary atom in the molecule constituting the chemical substance is a carbon atom on an aromatic ring in a mono-substituted benzenic compound, and the kind of activity that is to be predicted is orientation of nitration.

9. The prediction method according to claim 2 or 3, wherein the arbitrary atom in the molecule constituting the chemical substance is a carbon atom at ortho or para position in biphenyl, and the kind of activity that is to be predicted is "ortho/para orientation in electrophilic substitution reaction of biphenyl".

FIG.1

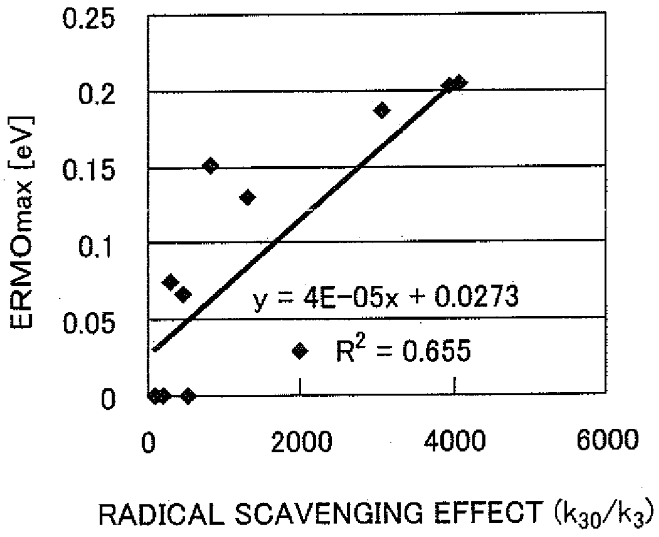
0.25
0.2
0.15
0.1
0.05
0
ERMOmax [eV]
y = 4E-05x + 0.0273
R2 = 0.655
0
2000
4000
6000
RADICAL SCAVENGING EFFECT (k30/k3)

FIG.2

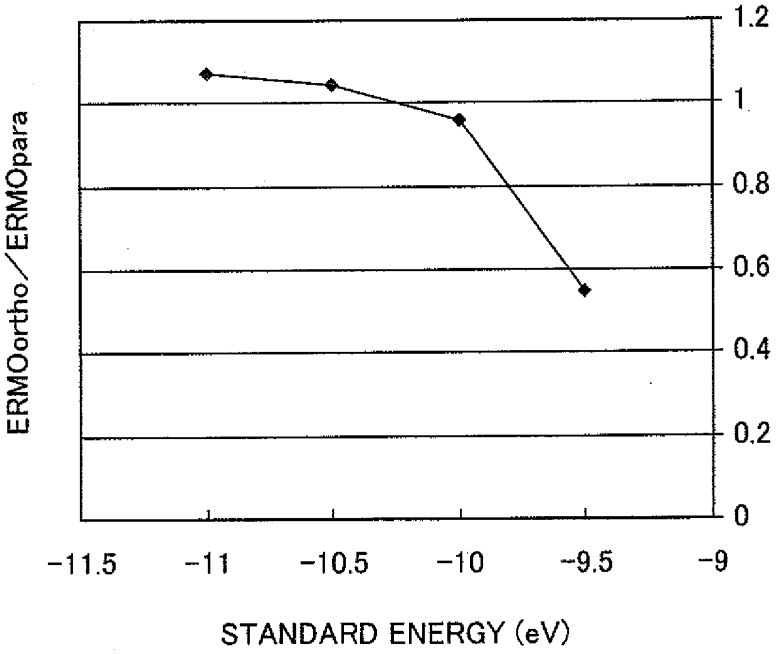
1.2
1
0.8
0.6
0.4
0.2
0
ERMOortho／ERMOpara
−11.5
−11
−10.5
−10
−9.5
−9
STANDARD ENERGY (eV)

**INTERNATIONAL SEARCH REPORT**

International application No.
PCT/JP2011/054681

A. CLASSIFICATION OF SUBJECT MATTER
*C07B61/00*(2006.01)i, *G01N33/15*(2006.01)n

According to International Patent Classification (IPC) or to both national classification and IPC

B. FIELDS SEARCHED

Minimum documentation searched (classification system followed by classification symbols)
C07B61/00, G01N33/00, G06F15/00, G06F17/00, G06F19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 11-287800 A (Sumitomo Chemical Co., Ltd.), 19 October 1999 (19.10.1999), full specifications (Family: none) | 1-9 |
| Y | Yasukazu OHKATSU, Shin-ichi ISHIKAWA, Etsuo TOBITA, Consideration on the effect of ortho-substituents of phenols by semiempirical molecular orbital method MOPAC, Polymer Degradation and Stability, 2000, Vol.67, p.541-545 | 1-9 |
| Y | JP 2001-278859 A (Mitsui Chemicals, Inc.), 10 October 2001 (10.10.2001), paragraphs [0005] to [0006] (Family: none) | 1-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>23 March, 2011 (23.03.11) | Date of mailing of the international search report<br>05 April, 2011 (05.04.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/054681

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-2238 A (Celestar Lexico-Sciences, Inc.), 08 January 2004 (08.01.2004), full specifications & US 2005/0130224 A1 & EP 1510943 A1 & WO 2003/107218 A1 | 1-9 |
| Y | JP 2003-183186 A (Canon Inc.), 03 July 2003 (03.07.2003), full specifications (Family: none) | 1-9 |
| A | JP 2000-143554 A (The Institute of Physical and Chemical Research), 23 May 2000 (23.05.2000), full specifications & US 6839634 B1 | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004012451 A


### Non-patent literature cited in the description


- **PATLEWICZ G. et al.** *Chem. Res. Toxicol.,* 2008, vol. 21, 521
- **ROBERTS D. W.** *Chem. Res. Toxicol.,* 1995, vol. 8, 545
- **TOMLIN C. D. S.** The Pesticide Manual. British Crop Protection Council, 1997
- **KEI MATSUZAKI ; ZENJIRO OSAWA.** Automatic Oxidization (Chemical Mechanism and Application. MARUZEN, 1972, 159